# EUROPÄISCHE PATENTANMELDUNG

(11) **EP 2 292 117 A1**
(43) Veröffentlichungstag der Anmeldung: **09.03.2011**
(21) Anmeldenummer: 10008073.8
(22) Anmeldetag: 03.08.2010
(51) Int. Cl.: A45F 3/14

(54) **Gürteltragevorrichtung für ein Kunstherzsystem**

(30) Priorität: 28.08.2009 DE 202009011683 U
(71) Anmelder: Schmidt, Antje, 32584 Löhne (DE)
(72) Erfinder: Schmidt, Antje, 32584 Löhne (DE)
(74) Vertreter: Brandt, Detlef

(57) **Zusammenfassung**

Es wird eine Gürteltragevorrichtung für ein Kunstherzsystem mit einer Aufnahme-vorrichtung (1) für eine Steuereinheit und mindestens einer Haltevorrichtung (2 u.3) für eine Akkueinheit zur Stromversorgung der Steuereinheit, wobei Aufnahmevorrichtung (1) und Haltevorrichtung (2 u. 3) an einem von einem Patienten umzulegenden Taillengürtel (4) festgelegt sind, vorgestellt, bei der erfindungsgemäß die Haltevorrichtung (2 u. 3) und Aufnahmevorrichtung (1) nebeneinander am Taillengürtel (4) angeordnet sind und bei der die Breite des Taillengürtels (4) mindestens die Höhe der Aufnahmevorrichtung (1) für die Steuereinheit umfasst, wobei die gesamte zum Taillengürtel (4) gerichtete Rückwand (6) der Aufnahmevorrichtung (1) fest mit dem Taillengürtel (4) verbunden ist, aufweist.

## Beschreibung

### Technisches Umfeld

Die Erfindung betrifft eine Gürteltragevorrichtung für ein Kunstherzsystem mit einer Aufnahmevorrichtung für eine Steuereinheit und mindestens einer Haltevorrichtung für eine Akkueinheit zur Stromversorgung der Steuereinheit, wobei Aufnahmevorrichtung und Haltevorrichtung an einem von einem Patienten umzulegenden Taillengürtel festgelegt sind.

### Stand der Technik

Auf Grund bedeutender Fortschritte in der Medizintechnik werden seit einiger Zeit vermehrt Herzoperationen durchgeführt, bei denen einem Patienten ein Kunstherzsystem eingesetzt wird. Dieses Kunstherzsystem ist notwendigerweise mit einer außerhalb des Patientenkörpers befindlichen Steuereinheit über eine sogenannte Driveline verbunden. Die Steuereinheit wiederum wird durch mindestens eine Akkueinheit mit elektrischer Energie versorgt. Das Gesamtgewicht der genannten Komponenten liegt üblicherweise bei mehr als zwei Kilogramm. Zur Aufbewahrung und zum Transport der außerhalb des Patientenkörper befindlichen Komponenten des Kunstherzsystems sind im Stand der Technik Tragetaschen bekannt, die vom Patienten auf unterschiedliche Weise am Körper mitgeführt werden.

Zum einen besteht bei den üblichen Tragetaschen die Möglichkeit, diese mittels eines über die Schulter gelegten Gurtes zu transportieren. Eine derartige Trageweise belastet naturgemäß die Schultern des Patienten nicht unerheblich, deshalb ist durch die einseitige Belastung einer Schulter gerade unter der Voraussetzung des notwendigen permanenten Tragens der Tasche auf längere Sicht mit erheblichen Verspannungen und Fehlhaltungen beim Patienten zu rechnen. Darüber hinaus besteht bei einem Umhängen der Tasche die permanente Gefahr des Hängenbleibens mit dem Schultergurt beispielsweise an Stuhllehnen oder an Türklinken. Naturgemäß ist in heutiger Zeit auch die Gefahr des Entreißens durch Taschenräuber gegeben.

Aus den oben genannten Gründen bevorzugen einige Patienten die Variante, die Tragetasche an einem herkömmlichen Gürtel um die Hüfte zu tragen. Hierzu wird die Tasche mittels eines Klettverschlusses oder entsprechender Karabinerhaken am Gürtel befestigt. Da die aus dem Stand der Medizintechnik bekannten Tragetaschen eine Anordnung der Steuereinheit zusammen mit einem oder zwei Akkueinheiten hintereinander in einer gemeinsamen Tragetascheneinheit vorsehen, hängt die am Gürtel getragene Tragetasche auch auf Grund der lockeren Befestigung am Gürtel stark nach unten durch. Durch diese Tatsache sowie durch die ausladende Plazierung von Steuereinheit und Energieversorgungseinheiten stört die Gesamtanordnung z. B. beim Gehen, beim Anschnallen im Auto oder beim Toilettengang, aber auch bei sportliche Aktivitäten wie Rad fahren oder Ski laufen.

### Aufgabe der Erfindung

Aufgabe der vorliegenden Erfindung ist es deshalb, ausgehend von den aus dem Stand der Technik bekannten Tragemöglichkeiten für ein Kunstherzsystem eine medizinisch vorteilhafte und patientenfreundliche Möglichkeit bereitzustellen, um die außerhalb des Körpers mitzuführenden Bestandteile des Kunstherzsystems in Form einer Steuereinheit und einer oder mehrerer Akkueinheiten vorteilhaft ohne dauerhafte medizinische Beeinträchtigung zu transportieren.

### Lösung der Aufgabe

Die gestellte Aufgabe wird für eine Gürteltragevorrichtung für ein Kunstherzsystem mit den oben angeführten gattungsbildenden Merkmalen durch die im kennzeichnenden Teil des Anspruches 1 offenbarte technische Lehre gelöst.

Erfindungswesentüch dabei ist es, dass die Aufnahmevorrichtung für die Steuereinheit und die Hattevorrichtung für die Akkueinheit nebeneinander am Taillengürtel angeordnet sind und dass die Breite des Taillengürtels mindestens die Höhe der Aufnahmevorrichtung für die Steuereinheit umfasst, wobei die gesamte, zum Taillengürtel gerichtete Rückseite der Aufnahmevorrichtung fest mit dem Taillengürtel verbunden ist.

Durch diese Gesamtkombination der Merkmale entsteht eine kompakte beim Tragen an der Hüfte wenig ausladende Einheit, die auf Grund der Konzeption des Taillengürtels für den Patienten einen außerordentlich angenehmen Tragekomfort bietet. Darüber hinaus sind alle außerhalb des Körpers befindlichen Komponenten des Kunstherzsystems dicht am Körper festgelegt, so dass ein Durchhängen der Gürteltragevorrichtung ebenso wie körperliche Fehlhaltungen vermieden werden. Durch die Erfindung wird somit den Kunstherzpatienten die Teilnahme am Alltagsleben wesentlich erleichtert. Darüberhinaus kann durch die eng am Körper platzierten Komponenten des Kunstherzsystems vorn Patienten eine schlaufenfreie Verlegung der Verkabelung sowie der Driveline vorgenommen werden.

Besondere Weiterbildungen des Gegenstandes der Erfindung sind darüber hinaus in den auf den Anspruch 1 rückbezogenen Unteransprüchen offenbart.

Es hat sich insbesondere als vorteilhaft erwiesen, wenn die Aufnahmevorrichtung für die Steuereinheit eine Rückwand, eine Vorderwand, Seitenwände und einen Deckel aufweist, die durch Verschlusselemente zu einer geschlossenen Tasche koppelbar sind und die im entkoppelten Zustand in die Lageebene der Rückwand klappbar sind.

Durch diese Maßnahme ist ein problemloses Einsetzen der Steuereinheit in die Aufnahmevorrichtung gewährieistet, da wie oben eingangs bereits ausgeführt, eine ständige Verbindung zwischen Steuereinheit und Kunstherz durch die Driveline gewährleistet sein muss, eine Abkoppelung der Driveline zum Einführen der Steuereinheit in die Aufnahmevorrichtung also unter allen Umständen zu vermeiden ist.

Darüber hinaus ist es notwendig, die Steuereinheit gleichzeitig immer mit einer Akkueinheit in Verbindung stehen zu lassen, um die ununterbrochene Energieversorgung zu gewährleisten. Da die Anschlüsse für die Akkueinheit und zur Driveline bei der Steuereinheit aus konstruktiven Gründen an unterschiedlichen Seiten angeordnet ist, kann das Aufklappen und Flachlegen der zur Aufnahmevorrichtung gehörenden Wände und des Deckels die Handhabung der erfindungsgemäßen Gürtehragevorrichtung wesentlich erleichtern.

Aus fertigungstechnische Gründen kann es darüber hinaus vorteilhaft sein, wenn Vorderwand, Rückwand, Seitenwände und Deckel der Aufnahmevorrichtung einstückig ausgebildet sind.

Zusätzlich hat es sich als vorteilhaft erwiesen, wenn die an der Aufnahmevorrichtung, der Haltevorrichtung für die Akkueinheit sowie am Taillengürtel vorhandenen Verschlusselemente jeweils redundant zweifache Koppelvorrichtungen aufweisen. Auf diese Weise ist die aus medizinischer Hinsicht notwendige Sicherheit beim Tragen der Gürteltragevorrichtung jederzeit vorhanden.

Die erste Koppelvorrichtung kann dabei beispielsweise als Klettverschluss ausgebildet sein, wohingegen die zweite Koppelvorrichtung als Schnappverschluss ausgebildet wird. Die Schnappverschlüsse am Erfindungsgegenstand sind dabei vorteilhafterweise aus besonders belastbaren sowie hitze- und kältebeständigen Kunststoffmaterial gefertigt, so dass sowohl im Winter als auch im Sommer bei entsprechenden Außentemperaturen die Patienten aufgrund der ausreichenden Bruchfestigkeit eine Beschädigung der Kunststoffelemente des Schnappverschlusses aufgrund von Umwelteinflüssen nicht befürchten müssen.

Zur Erhöhung des Tragekomforts ist entsprechend einer weiteren vorteilhaften Ausgestaltung die Länge des Taillengortels einstellbar. Zusätzlich kann zur Erweiterung der Gürtellänge insbesondere bei Patienten mit sehr grossen Taillenumfang ein Zwischenglied zwischen die Enden des Taillengürtels eingesetzt werden. Dieses Zwischenglied ist beidseitig an die jeweiligen sich am Ende des Taillengürtels befindlichen Verschlusselemente derart angepasst, dass die entsprechenden Elemente der Schnappverschlüsse und Kiettverschlüsse genutzt werden können, um das Zwischenglied dazwischen einzupassen.

Da für eine permanente Energieversorgung des Kunstherzsystems vorteilhafterweise mindestens zwei Akkueinheiten verwendet werden, hat es sich darüber hinaus als vorteilhaft erwiesen, die dafür notwendigen zwei Haltevorrichtungen für je eine Akkueinheit rechts und links der Ausnahmevorrichtung für die Steuereinheit anzuordnen. Diese Maßnahme führt zu einer Verbesserung der Gewichtsverteilung an der Gürteltragevorrichtung.

Zur Erleichterung der Handhabung kann es darüber hinaus zweckmäßig sein die Aufnahmevorrichtung und die Haltevorrichtungen mit Ausnehmungen zur Durchführung von Kabelleitungen wie beispielsweise der Driveline oder Leitungen zur Stromversorgung zu versehen.

Um die Steuereinheit einfacher überwachen zu können, kann es darüber hinaus vorteilhaft sein, wenn der Deckel der Aufnahmevorrichtung für die Steuereinheit ein Sichtfenster zur Beobachtung eines Displays der Steuereinheit aufweist. Zweckmäßig ist überdem, wenn die Vorderseite der Aufnahmevorrichtung für die Steuereinheit eine Öffnung für die Ausgabe von Tönen eines Signalgebers der Steuereinheit ausweist.

### Figurenbeschreibung

Anhand der beigefügte Figuren wird nachfolgend ein Ausführungsbeispiel des Gegenstandes der Erfindung näher erläutert.

Es zeigt
- Figur 1: eine perspektivische Ansicht der erfindungsgemäßen Gürteltrage- vorrichtung und
- Figur 2: eine Ansicht der Gürteltragevorrichtung mit geöffneter Aufnahme- Vorrichtung.

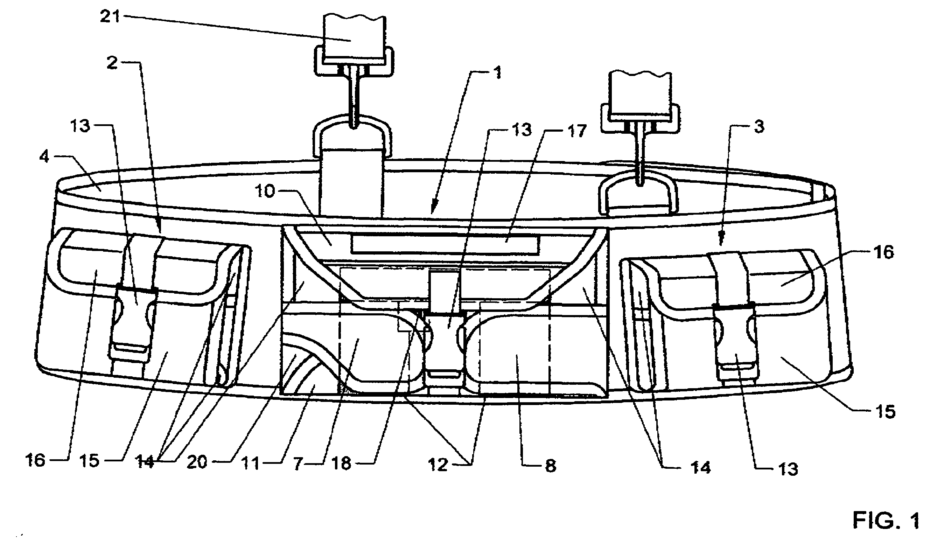

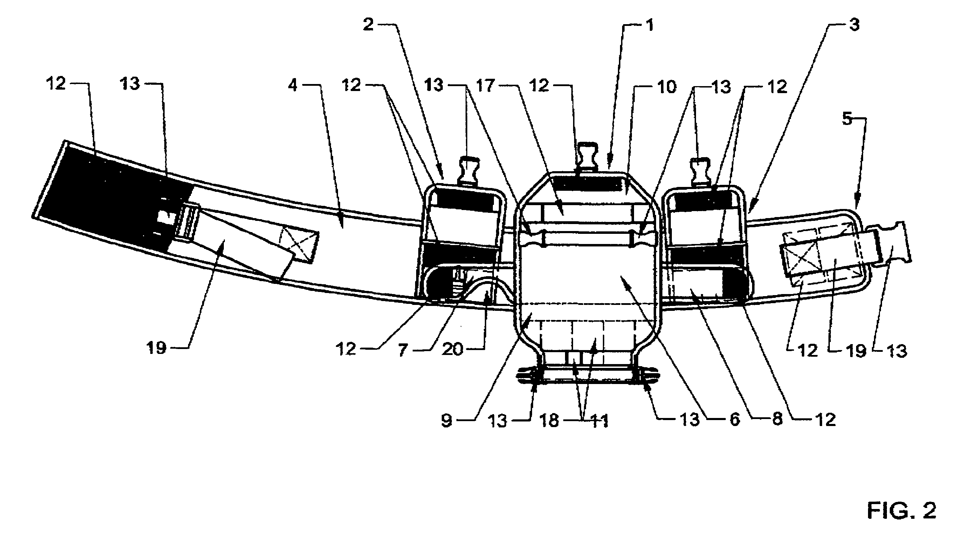

Die in den Figuren 1 und 2 dargestellte erfindungsgemäße Gürteltragevorrichtung besteht im Wesentlichen aus einem Taillengürtel 4, einer Aufnahmevorrichtung 1 für die Steuereinheit eines Kunstherzsystems sowie zwei Haltevorrichtungen 2, 3 jeweils für Akkueinheiten zur elektrischen Energieversorgung der Steuereinheit sowie des Kunstherzes.

Aus Gründen der Übersichtlichkeit wurde in den Figuren auf die Darstellung der Steuereinheit sowie der Akkueinheiten verzichtet. Wie aus den Figuren ersichtlich ist, befinden sich die Haltevorrichtungen 2 und 3 jeweils rechts und links der Aufnahmevorrichtung 1. Sie sind unmittelbar benachbart angeordnet, so dass zwischen Akkueinheiten und der mittig liegenden Steuereinheit kurze Kabelwege verwirklicht werden können. Der Taillengürtel 4 ist erfindungsgemäß so ausgestaltet, dass er mindestens die Höhe der Aufnahmevorrichtung 1 für die Steuereinheit aufweist. Die gesamte zum Teillengürtel 4 gerichtete Rückwand 6 der Aufnahmevorrichtung 1 ist dabei fest mit dem Taillengürtel 4 verbunden. Auf diese Weise wird eine Relativbewegung zwischen Aufnahmevorrichtung 1 und Taillengürtel 4 verhindert.

Aus der Figur 1 ist darüber hinaus ersichtlich, dass auch die Rückseite der Haltevorrichtungen 2 und 3 ganzflächig mit dem Taillengürtel 4 verbunden sind, so dass auch hier eine kompakte bewegungsfreie Einheit zwischen Taillengürtel 4 und Haltevorrichtungen 2 und 3 entsteht.

Die aus Gründen der Übersichtlichkeit nicht dargestellte Steuereinheit ist üblicherweise durch eine sogenannte Driveline mit dem im Körper des Kunstherzpatienten befindlichen Kunstherz verbunden. Gleichzeitig muss die Steuereinheit immer mit mindestens einer Akkueinheit verbunden sein. Aus diesem Grunde ist entsprechend einer vorteilhaften Weiterbildung des Erfindungsgegenstandes die Aufnahmevorrichtung 1 speziell gestaltet.

Sie weist eine fest mit dem Taillengürtel verbundene Rückwand 6 auf, Seitenwände 7, 8 sowie eine Seitenwand 9 als Boden der Aufnahmevorrichtung. Darüber hinaus besitzt die Aufnahmevorrichtung eine Vorderwand 11, sowie ein Deckel 10. Sämtliche oben genannten Bestandteile der Aufnahmevorrichtung 1 sind im vorliegenden Ausführungsbeispiel einstückig aus einem Teil gefertigt und mit dem Taillengürtel 4 verbunden. Durch diese Ausgestaltung kann die Aufnahmevorrichtung zum einen durch Verschlusselemente zu einer geschlossene Tasche gekoppelt werden, im entkoppelten Zustand der Bestandteile sind die Seitenwände 7, 8, 9, der Deckel 10 sowie die Vorderwand 11 jedoch in die Lageebene der Rückwand 6 klappbar, so dass die aufzunehmende Steuereinheit problemlos an die Rückwand 6 angelegt und die Aufnahmevorrichtung dann geschlossen werden kann.

Zu diesem Zweck werden zunächst die Seitenwand 9 als Boden mit der Vorderwand 11 nach oben in Richtung des Deckels 10 geklappt und danach die Seitenwand 7 und 8 über die Vorderwand 11 geschlagen. Die Seitenwand 7 und 8 werden jeweils durch Koppelvorrichtungen 12 in Form von Klettverschlüssen und Koppelvorrichtungen 13 in Form von Schnappverschlüssen mit der Vorderwand 11 verbunden. Zur fertigen Formgebung der Tasche für die Steuereinheit wird dann der Deckel 10 vom Taillengürtel 4 weg nach vorne geklappt, so dass eine geschlossene Tasche entsteht. Der Deckel 10 wird ebenso wie die Seitenwände mit der Vorderwand durch jeweils eine Koppelvorrichtung 12 In Form eines Klettverschlusses sowie eine Koppelvorrichtung 13 in Form eines Schnappverschlusses fixiert.

Der Taillengürtel 4 selbst ist zur Anpassung an die körperlichen Gegebenheiten des Nutzers in seiner Länge einstellbar, wobei die beiden Enden des Taillengürtels mittels eines Gürtelverschlusses 5 verbunden werden, der ebenfalls zwei Koppelvorrichtungen 12 und 13 in Form eines Klettverschlusses sowie eines Schnappverschlusses aufweist. Die Schließmechanismen des Schnappverschlusses sind zur Längeneinstellung des Taillengürtels an Bändern 19 befestigt.

Wird von dem Patienten als zusätzliche Sicherung ein Schultergurt 21 gewünscht, so kann dieser wie in der Figur 1 gezeigt zusätzlich am Taillengürtel mittels geeigneter Ankoppelungsmöglichkeiten festgelegt werden.

Die Haltevorrichtungen 2 und 3 für die Akkueinheiten sind im Gegensatz zur Aufnahmevorrichtung 1 als geschlossene Akkutaschen 15 ausgebildet und lediglich an ihrer Oberseite durch einen Akkutaschendeckel 16 zu öffnen. Die lösbare Verbindung zwischen Akkutaschendeckel 16 und der Vorderseite der Akkutasche 15 ist wie oben bereits in anderem Zusammenhang beschrieben redundant durch zwei Koppelvorrichtungen realisiert, wobei eine Koppelvorrichtung 12 als Klettverschluss und die andere Koppelvorrichtung 13 als Schnappverschluss ausgestaltet sind. Im Bereich zwischen Akkutaschendeckel 16 und Akkutasche 15 ist jeweils eine Ausnehmung für eine Kabelleitung vorhanden, wobei die Verbindung zwischen Akkueinheit und Steuereinheit im Bereich der Aufnahmevorrichtung 1 ebenfalls durch eine zwischen den Seitenwanden 7, 8 und dem Deckel 10 der Aufnahmevorrichtung vorhandene Ausnehmung 14 möglich ist.

Im Bereich der Seitenwand 7 ist darüber hinaus an der zum Boden gerichtete Unterseite eine weitere Ausnehmung 20 vorhanden, durch welche die oben genannte Driveline für die Verbindung zum Kunstherz durchgeführt werden kann.

Aus der Figur 1 und 2 ist darüber hinaus ersichtlich, dass im Deckel 10 der Aufnahmevorrichtung 1 ein Sichtfenster 17 eingebracht ist, welches mit einer durchsichtigen Folie versehen sein kann und die Möglichkeit der Beobachtung eines an der Oberseite der Steuereinheit vorhandenen Display ermöglicht. An der Vorderwand 11 weist die Aufnahmevorrichtung darüber hinaus eine zusätzliche Öffnung 18 auf, durch die Töne eines Signalgebers der Steuereinheit nach außen dringen können.

Sämtliche Schnappverschlüsse der Koppelvorrichtungen 13 an den verschiedenen Bestandteilen der Gürteltragevorrichtung sind aus besonders bruchfesten sowie hitze- und kältebeständigem Kunststoff gefertigt, so dass auch bei erhöhten oder niedrigen Temperaturen eine Beschädigung der Kunststoffteile vermieden wird.

Durch die erfindungsgemäße Gestaltung wird erstmals eine allen medizinischen Anforderungen gerecht werdende Transportmöglichkeit für die außerhalb des Körpers eines Kunstherzpatienten befindlichen Bestandteile eines Kunstherzsystems bereit gestellt. Gleichzeitig weist die Erfindung gegenüber herkömmlichen Tragemöglichkeiten signifikante Vorteile im Hinblick auf ein erhöhten Tragekomfort sowie die Verhinderung von tragebedingten Gesundheitsbeeinträchtigungen auf. Dem Kunstherzpatienten wird somit die Teilnahme am täglichen Leben wesentlich erleichtert. Darüberhinaus sind auch sportliche Aktivitäten beispielsweise zur Rehabilitation wie Rad fahren oder Ski laufen problemlos möglich, da die Arme des Patienten vollkommen frei beweglich sind.

### Bezugseichnungsliste

- 1: Aufnahmevorrichtung
- 2: Haltevorrichtung
- 3: Haltevorrichtung
- 4: Taillengürtel
- 5: Gürtelverschluss
- 6: Rückwand
- 7: Seitenwand
- 8: Seitenwand
- 9: Seitenwand
- 10: Deckel
- 11: Vorderwand
- 12: Koppelvorrichtung Klettverschluss
- 13: Koppelvorrichtung Schnappversohluss
- 14: Ausnehmung für Kabelleitung
- 15: Akkutasche
- 16: Akkutaschendeckel
- 17: Sichtfenster
- 18: Öffnung
- 19: Band
- 20: Ausnehmung
- 21: Schultergurt

## Patentansprüche

1. Gürteltragevorrichtung für ein Kunstherzsystem mit einer Aufnahmevorrichtung (1) für eine Steuereinheit und mindestens einer Haltevorrichtung (2, 3) für eine Akkueinheit zur Stromversorgung der Steuereinheit, wobei Aufnahmevorrichtung (1) und Haltevorrichtung (2, 3) an einem von einem Patienten umzulegenden Taillengürtel (4) festgelegt sind,
**dadurch gekennzeichnet, dass**
Haltevorrichtung (2, 3) und Aufnahmevorrichtung (1) nebeneinander am Taillengortel (4) angeordnet sind und dass die Breite des Taillengürtels (4) mindestens die Höhe der Aufnahmevorrichtung (1) für die Steuereinheit umfasst und wobei die gesamte zum Taillengürtel (4) gerichtete Rückwand (6) der Aufnahmevorrichtung (1) fest mit dem Taillengürtel (4) verbunden ist.

2. Gürteltragevorrichtung nach Anspruch 1,
**dadurch gekennzeichnet, dass**
die Aufnahmevorrichtung (1) für die Steuereinheit eine Rückwand (6), eine Vorderwand (11), Seitenwände (7, 8, 9) und einen Deckel (10) aufweist, die durch Verschlusselemente zu einer geschlossenen Tasche koppelbar sind und im entkoppelten Zustand in die Lageebene der Rückwand (6) klappbar sind.

3. Gürteltragevorrichtung nach Anspruch 2,
**dadurch gekennzeichnet, dass**
die Vorderwand (11), Rückwand (6), Seitenwände (7, 8, 9) und Deckel (10) einstückig ausgebildet sind.

4. Gürteltragevorrichtung nach einem der Ansprüche 2 oder 3,
**dadurch gekennzeichnet, dass**
die Verschlusselemente jeweils redundant zweifache Koppelvorrichtungen (12 u 13)aufweisen.

5. Gürteltragevorrichtung einem der Ansprüche 1 bis 4
**dadurch gekennzeichnet, dass**
der Gürtelverschluss (5) redundant zwei Koppelvorrichtungen (12 u 13) aufweist.

6. Gürteltragevorrichtung nach einem der Ansprüche 2 bis 5,
**dadurch gekennzeichnet, dass**
eine Koppelvorrichtung als Klethrerschluss (12) ausgebildet ist.

7. Gorteltragevorrichtung nach Anspruch 4 oder 5,
**dadurch gekennzeichnet, dass**
die zweite Koppelvorrichtung als Schnappverschluss (13) ausgebildet ist.

8. Gürteltragevorrichtung nach einem der Ansprüche 1 bis 7,
**dadurch gekennzeichnet, dass**
die Schnappverschlüsse (13) aus hitze- und kältebeständigem Kunststoffmaterial gefertigt sind.

9. Gürteltragevorrichtung nach einem der Ansprüche 1 bis 8,
**dadurch gekennzeichnet, dass**
die Länge des Taillengürtels (4) einstellbar ist

10. Gürteltragevorrichtung nach einem der Ansprüche 1 bis 9,
**dadurch gekennzeichnet, dass**
zwei Haltevorrichtungen für je eine Akkueinheit rechts und links der Aufnahmevorrichtung für die Steuereinheit angeordnet sind.

11. Gürteltragevorrichtung nach einem der Ansprüche 1 bis 10,
**dadurch gekennzeichnet, dass**
die Aufnahmevorrichtung (1) und die Haltevorrichtung (2) Ausnehmungen (14, 20) zur Durchführung von Kabelleitungen ausweisen.

12. Gürteltragevorrichtung nach einem der Ansprüche 1 bis 11,
**dadurch gekennzeichnet, dass**
der Deckel der Aufnahmevorrichtung (1) für die Steuereinheit ein Sichtfenster (17) zur Beobachtung eines Displays der Steuereinheit aufweist.

13. Gürteltragevorrichtung nach einem der Ansprüche 1 bis 12,
**dadurch gekennzeichnet, dass**
die Vorderseite der Aufnahmevorrichtung (1) für die Steuereinheit eine Öffnung (18) für die Töne eines Signalgebers der Steuereinheit aufweist.
